(19) Europäisches Patentamt
European Patent Office
Office européen des brevets

(11) **EP 4 220 665 A1**

(12) **EUROPEAN PATENT APPLICATION**

(43) Date of publication:
**02.08.2023 Bulletin 2023/31**

(21) Application number: **23151862.2**

(22) Date of filing: **17.01.2023**

(51) International Patent Classification (IPC):
**G16H 50/30** (2018.01)     **G16H 40/63** (2018.01)
**G01G 1/00** (2006.01)     **A61B 5/00** (2006.01)
**A61B 5/0537** (2021.01)     **G01G 19/50** (2006.01)

(52) Cooperative Patent Classification (CPC):
**G16H 40/63; A61B 5/0537; A61B 5/4869;
G16H 50/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.01.2022 TW 111104029**

(71) Applicant: **Starbia Meditek Co., Ltd.
Taichung City 402 (TW)**

(72) Inventors:
• **HSIEH, KUEN-CHANG**
 **402 Taichung City (TW)**
• **TSAI, CHIH-CHING**
 **402 Taichung City (TW)**
• **LIN, SHIN-TA**
 **402 Taichung City (TW)**

(74) Representative: **Straus, Alexander
2K Patent- und Rechtsanwälte - München
Keltenring 9
82041 Oberhaching (DE)**

(54) **DATA INTEGRATION SYSTEM OF BODY COMPOSITION ANALYZER**

(57)     A data integration system (10) of a body composition analyzer includes a high-precision body composition analyzer (20), a low-precision body composition analyzer (30), and a computing device (40). The computing device (40) is used to calculate a deviation value between the high-precision body composition analyzer (20) and the low-precision body composition analyzer (30), and correct the data generated by the low-precision body composition analyzer (30) through the deviation value so as to obtain more accurate body composition data.

FIG. 1

**EP 4 220 665 A1**

**Description**

**BACKGROUND OF THE INVENTION**

**1. Field of the Invention**

The present invention relates to body composition analysis techniques and more particularly, to a data integration system of a body composition analyzer.

**2. Description of the Related Art**

As living standards improve, people's demand for maintaining good health is also increasing day by day. Health is closely related to an individual's body composition. A normal and balanced body composition is one of the basic conditions for maintaining good health. Body composition can be measured by a plurality of different manners, including commonly used manners such as imaging manners and bioelectrical impedance analysis (BIA). The more commonly used imaging manner includes computed tomography (CT), magnetic resonance imaging (MRI), and dual-energy X-ray absorptiometry (DXA). Because DXA has high stability and accuracy, and has lower cost than that of CT and MRI, DXA has become a gold standard for body composition measurement. BIA has advantages of convenient use, fast operation, low cost, safe and non-invasive manner, and acceptable accuracy, so it has become one of the most widely used manners, and further, BIA products have been popularized, and the market continues to develop rapidly. In addition to weight, BIA products provide important parameters such as lean body mass, body fat weight, body fat percentage, muscle mass, and total body water.

Body composition analysis products on the market can be roughly divided into two types: an advanced professional type and a household type. The advanced professional-type machines usually use multi-frequency alternating current. The most common multi-frequency BIA products, its operating frequency is mostly three frequencies to six frequencies. The impedance value and phase angle of the whole body or different limbs can be obtained by using eight electrode plates and multi-frequency multi-limb measurement technology. Finally, dozens of body composition parameters can be obtained by substituting the measured impedance value of each limb and the user's personal information such as height, age, weight, gender, race, and exercise habits, etc., in the estimation equation. The correctness of the above estimation equation will be verified by the test results of DXA or CT. Many researches have shown that compared with the results obtained by DXA, the professional-type machines of various brands have very high correlation with the estimation value of body fat percentage and muscle mass of general users, although there are deviations in the estimation value. Some products are even as high as 0.97 to 0.98, and only special groups such as those who are extremely thin, extremely obese or have diseases will have a larger error. Therefore, the results obtained by the professional-type machines are very close to the medical level so as to have a relatively high reference value. However, in order to achieve high measurement accuracy, the professional-type machines are not only expensive, but also bulky, heavy and difficult to carry. They are usually only used in medical institutions and fitness industries.

On the contrary, the main purpose of the household-type body composition analyzers is to facilitate the use in daily life, so the design tends to be light and thin. Most of the early products were designed with four electrode plates, using a single-frequency 50kHz AC to perform foot-to-foot impedance measurement, that is, to estimate the body composition of the whole body only based on the impedance value of the legs. However, the proportion of leg muscles is quite high, and the torso, which accounts for the largest proportion of body weight, is not within the measurement range. The accuracy of such measurement has a certain gap with that of the advanced professional-type machines. In most reports, the household-type machines underestimate the fat mass and overestimate the muscle mass. Only a few test reports point out that the household-type machines overestimate the fat mass, which may be caused by different test groups. Generally speaking, the main difference between the household-type machine and the professional-type machine is that the estimated value of the household-type machine has a large error, and because of the use design, the standard deviation of the impedance measurement value of the household-type machine is relatively larger. Even if the result of the household-type machine has a certain deviation, and the accuracy of the household-type machine is not as good as that of the professional-type machine, years of researches have shown that regardless of the results of body fat or muscle mass estimation, the overall trend of the household-type machine is still highly correlated with the result of the professional-type machine, so using the household-type machine to track body composition parameters still has a certain reference value. At present, some household-type machines use eight electrode plates and dual-frequency measurement technology to make the estimated results closer to the estimated results of the professional-type machines. However, these machines are relatively expensive and each have a price between the price of the household-type machine and the professional-type machine. Not as popular as the lower-level household-type machine.

Regular monitoring of the body composition is very important for maintaining health. It is usually recommended that a person should take a daily or weekly measurement and record it. However, for general users, unless there is a special need for medical behavior, they will only take a more precise measurement every few months or even a year, and only use the household-type machine for monitoring at other times. Because the accuracy of the household-type machine is not as good as that of the professional-type machine, the users actually have no way to accurately grasp the changes in personal body composition.

## SUMMARY OF THE INVENTION

It is a primary objective of the present invention to provide a data integration system of a body composition analyzer, which can integrate body composition data measured by an advanced professional body composition analyzer and body composition data measured by a household body composition analyzer to allow a participant to obtain body composition data with the household body composition analyzer that is close to that obtained with the advanced professional body composition analyzer after performing a measurement with the advanced professional body composition analyzer, thereby achieving the effect of accurately and easily grasping the changes in individual skeletal muscle mass.

To attain the above objective, the data integration system of the present invention comprises a high-precision body composition analyzer for detecting a first body composition data of a participant, a low-precision body composition analyzer for detecting a second body composition data of the participant, and a computing device electrically connected with the high-precision body composition analyzer and the low-precision body composition analyzer and receiving the first body composition data and the second body composition data. The computing device includes a database storing the first and second body composition data, and a computing unit using the first body composition data as a standard value and calculating a deviation value between the first and second body composition data and correcting the first or second body composition data through the deviation value to obtain a corrected second body composition data.

It can be seen from the above that the data integration system of the present invention uses the deviation value between the first and second body composition data to correct the second body composition data, such that after performing a measurement with the high-precision body composition analyzer, the participant can obtain the body composition data with the low-precision body composition analyzer close to that obtained with the high-precision body composition analyzer, thereby achieving the effect of accurately and easily grasping the changes in individual skeletal muscle mass.

Other advantages and features of the present invention will be fully understood by reference to the following specification in conjunction with the accompanying drawings, in which like reference signs denote like components of structure.

## BRIEF DESCRIPTION OF THE DRAWINGS

FIG. 1 is a schematic drawing of the present invention.
FIG. 2 is a block diagram of the present invention.
FIG. 3 is a schematic drawing of the present invention, showing the first body composition data, the second body composition data, and the corrected second body composition data.
FIG. 4 is a schematic drawing of the present invention, showing other high-precision body composition analyzers, low-precision body composition analyzers, and computing devices.

## DETAILED DESCRIPTION OF THE INVENTION

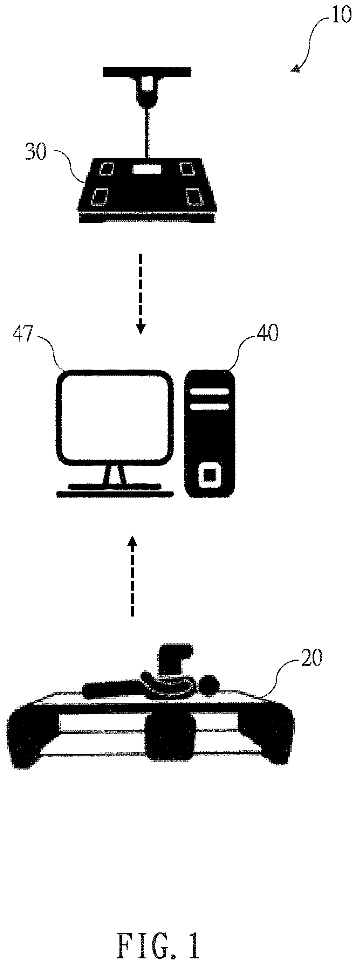

Referring to FIGS. 1 to 4, a data integration system 10 of a body composition analyzer of the present invention comprises a high-precision body composition analyzer 20, a low-precision body composition analyzer 30, and a computing device 40.

The high-precision body composition analyzer 20 is used to detect a first body composition data of a participant.

The low-precision body composition analyzer 30 is used to detect a second body composition data of the participant.

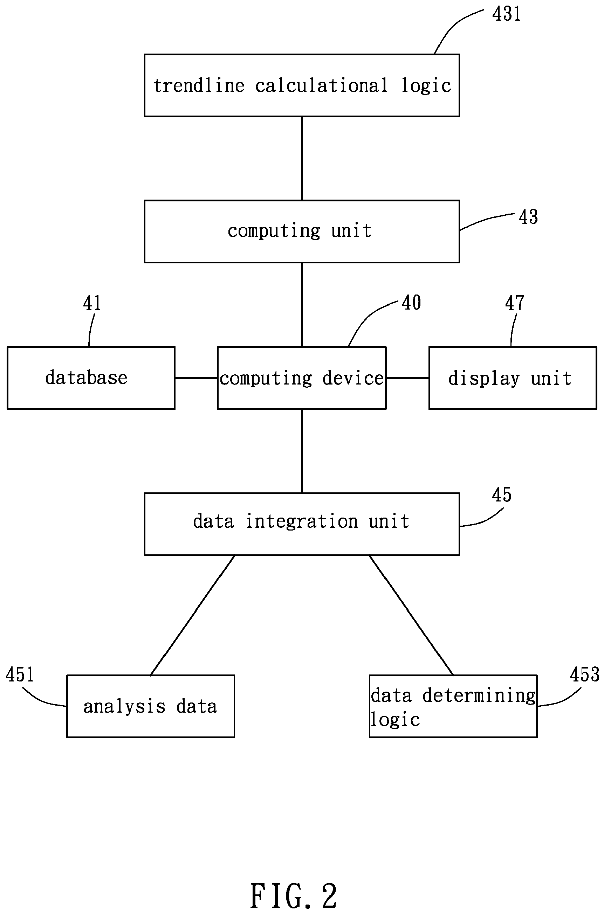

As shown in FIGS. 1 and 2, the computing device 40 is electrically connected with the high-precision body composition analyzer 20 and the low-precision body composition analyzer 30 and receives the first body composition data and the second body composition data. The computing device 40 includes a database 41 storing the first body composition data and the second body composition data, and a computing unit 43 using the first body composition data as a standard value and calculating a deviation value between the first body composition data and the second body composition data and correcting the second body composition data through the deviation value to obtain a corrected second body composition data.

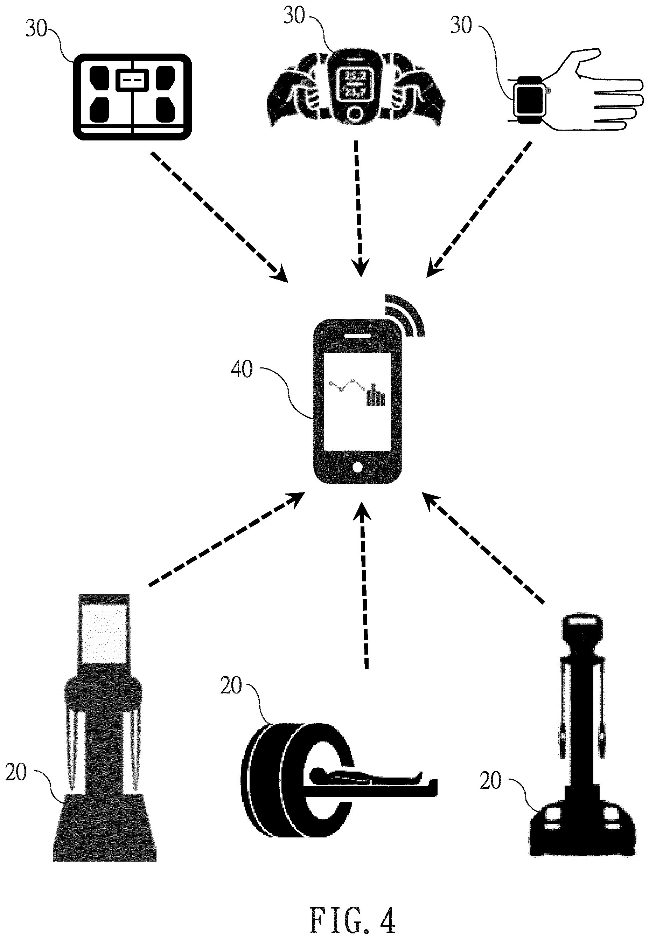

In this preferred embodiment, as shown in FIG. 1, a dual-energy Xray absorptiometry (DXA) is taken as the high-precision body composition analyzer 20, and a general household-type bioelectrical impedance analysis (BIA) is taken as the low-precision body composition analyzer 30, and a personal computer is taken as the computing unit 43. In other preferred embodiment, as shown in FIG. 4, the high-precision body composition analyzer 20 can be a computed tomography (CT) scanner, an advanced professional body composition analyzer or a magnetic resonance imaging (MRI) scanner (not shown). The low-precision body composition analyzer 30 can be other general household-type BIA (such as handheld type or wearable type). The computing device 40 can be a smartphone or tablet (not shown). Therefore, the high-precision body composition analyzer 20 and the computing device 40 are not limited to this preferred embodiment. Further, in other preferred embodiment, the high-precision body composition analyzer 20 and the low-precision body composition analyzer 30 can be plural in number and used by different participants. The first and second body composition data of the participants can be stored into the data base 41 and calculated through the computing unit 43. Therefore, the number of the high-precision body composition analyzer 20, the low-precision body composition analyzer 20, and the participant is not limited to this preferred embodiment.

In this preferred embodiment, the computing device 40 is electrically connected with the high-precision body composition analyzer 20 and the low-precision body composition analyzer 30 through a wireless connection (such as Bluetooth or Wi-Fi). In other preferred embodiment, the computing device 40 can be electrically connected with the high-precision body composition analyzer 20 and the low-precision body composition analyzer 30 through a wired connection. Therefore, the way of connecting the computing device 40 to the high-precision body composition analyzer 20 and the low-precision body composition analyzer 30 is not limited to this preferred embodiment.

In this preferred embodiment, the computing device 40 further includes a data integration unit 45 and a display unit 47. The data integration unit 45 requests the first and second body composition data from the database 41 and integrates the first and second body composition data into an analysis data 451 displayed on the displaying unit 47. The analysis data 451 can be presented according to actual needs, such as charts or text reports.

In this preferred embodiment, the first and second body composition data take skeletal muscle mass (SMM) as an example. When the first and second body composition data are plural in number, the corrected second body composition data is obtained by the following formula: $\widehat{x_B} = w_1 x_A + w_2(x_B - Bias_{BA})$,

where $\widehat{x_B}$ is the corrected second body composition data; $w_1 = \frac{\sigma_B^2}{\sigma_A^2 + \sigma_B^2}$ and $w_2 = \frac{\sigma_A^2}{\sigma_A^2 + \sigma_B^2}$ are weighting coefficients; $\sigma_A$ and $\sigma_B$ are standard deviations of the first and second body composition data, respectively; $\sigma_A$ is much smaller than $\sigma_B$; $Bias_{BA}$ is an average measurement deviation between the first and second body composition data. An initial default value for the average measurement deviation is zero. If $\sigma_A$ is zero and $\sigma_B$ is not zero, $\widehat{x_B} = x_A = x_B - (x_B - x_A)$ which is approximate to $\widehat{x_B} = x_A = x_B - Bias_{BA}$.

In other preferred embodiment, the first and second body composition data can be provided by BMI, fat and muscle assessment, obesity analysis, visceral fat, physical age or basal metabolic rate. The aforesaid formula can be changed according to the logic applied by the formula provided by this preferred embodiment. Therefore, the first and second body composition data are not limited to this preferred embodiment.

In this preferred embodiment, the data integration unit 45 further includes a data determining logic 453 requesting measurement time points of the plural first and second body composition data and classifying the first and second body composition data in pairs and obtaining plural data according the sequence of the measurement time points. When the plural data are zero, and there is no the average measurement deviation, the average measurement deviation is zero. When the plural data are one, and there is no the average measurement deviation, the average measurement deviation is a difference between the first and second body composition data. When the plural data are smaller than or equal to three, the average measurement deviation is based on those with a relatively large amount of usage data. When the plural data are larger than three, the average measurement deviation is obtained by using a statistical method. The statistical method can be performed by a multiple regression analysis or machine learning algorithm.

In this preferred embodiment, the data determining logic 453 includes, but not limited to, a predetermined interval capable of being set for requesting the measurement time points of the first and second body composition data within the predetermined range, thereby enhancing the authenticity of the average measurement deviation between the first and second body composition data.

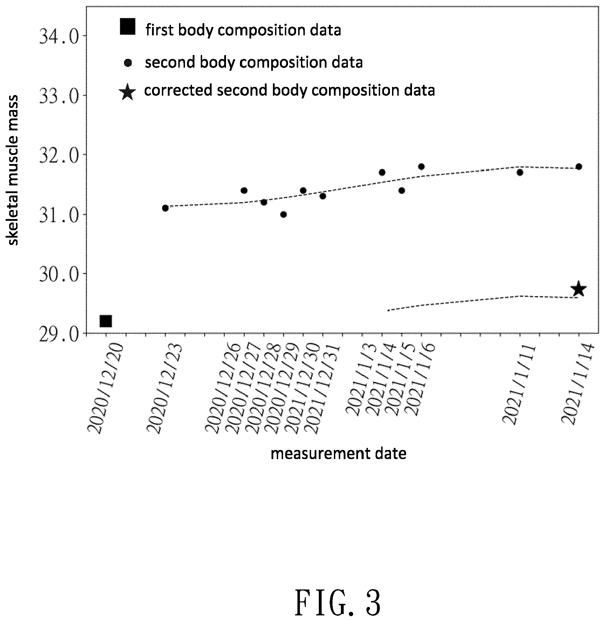

In this preferred embodiment, the computing unit 43 has a trendline calculational logic 431. When each of the first and second body composition data is one in number, the trendline calculational logic 431 is obtained by using a linear approximation (see the dotted line shown in FIG. 3). When each of the first and second body composition data are two in number, the trendline calculational logic 431 is obtained by using a non-linear approximation or piecewise linear regression analysis. In other preferred embodiment, the trendline calculational logic 431 can be obtained by Hilbert-Huang Transform. If the body composition data does not change much in a short period of time, it can be regarded as a steady-state time series. For example, intensive measurements in the short term can be obtained by Fourier transform. Therefore, the trendline calculational logic 431 is not limited to this preferred embodiment.

The above description is about the technical feature of this preferred embodiment, and the following description is about the measurement result of this preferred embodiment. For illustration purposes, the first and second body composition data of one participant are used to explain the measurement result.

As shown in FIG. 3, the data integration system 10 of the body composition analyzer of the present invention shows that the first body composition data (as shown in square) of the participant is detected by using the high-precision body composition analyzer 20 on December 20, 2020, and then a plurality the second body composition data (as shown in circles) of the participant are detected sequentially on December 23, 2020, December 26, 2020, December 27, 2020, etc. The corrected second body composition data (as shown in star shape) is obtained by the formula. Compared with the second body composition data and the corrected second body composition data detected on January 14, 2021, the skeletal muscle mass detected by the low-precision body composition analyzer 30 is about 31.7, but the skeletal muscle mass corrected by the present invention is about 29.7 that is close to the data measured by DXA.

As indicated above, the data integration system 10 of the body composition analyzer of the present invention uses the deviation value between the first and second body composition data to correct the second body composition data, such that after performing a measurement with the high-precision body composition analyzer 20, the participant can obtain the skeletal muscle mass with the low-precision body composition analyzer 30 that is close to the skeletal muscle mass obtained with the high-precision body composition analyzer 20, thereby achieving the effect of accurately and easily grasping the changes in individual skeletal muscle mass.

Other structures and attained effects of this second preferred embodiment are the same with those of the first preferred embodiment, thereby not repeatedly described.

## Claims

1. A data integration system (10) of a body composition analyzer, comprising:

   a high-precision body composition analyzer (20) for detecting a first body composition data of a participant;
   a low-precision body composition analyzer (30) for detecting a second body composition data of the participant; and
   a computing device (40) electrically connected with the high-precision body composition analyzer (20) and the low-precision body composition analyzer (30) and receiving the first body composition data and the second body composition data, the computing device (40) including a database (41) storing the first body composition data and the second body composition data, and a computing unit (43) using the first body composition data as a standard value and calculating a deviation value between the first body composition data and the second body composition data and correcting the first body composition data or the second body composition data through the deviation value to obtain a corrected second body composition data.

2. The data integration system (10) of the body composition analyzer as claimed in claim 1, wherein the computing device (40) is electrically connected with the high-precision body composition analyzer (20) and the low-precision body composition analyzer (30) through a wireless or wired connection.

3. The data integration system (10) of the body composition analyzer as claimed in claim 1, wherein the computing device (40) further comprises a data integration unit (45) requesting the first body composition data and the second body composition data from the database (41) and integrating the first body composition data and the second body composition data into an analysis data (451), and a display unit (47) displaying the analysis data (451) integrated by the data integration unit (45).

4. The data integration system (10) of the body composition analyzer as claimed in claim 1, wherein the first and second body composition data are skeletal muscle mass; when the first and second body composition data are plural in number, the corrected second body composition data is obtained by the following formula:

$$\widehat{x_B} = w_1 x_A + w_2 (x_B - Bias_{BA})$$

, where $\widehat{x_B}$ is the corrected second body composition data;

$$w_1 = \frac{\sigma_B^2}{\sigma_A^2 + \sigma_B^2} \quad \text{and} \quad w_2 = \frac{\sigma_A^2}{\sigma_A^2 + \sigma_B^2}$$

are weighting coefficients; $\sigma_A$ and $\sigma_B$ are standard deviations of the first and second body composition data, respectively; $\sigma_A$ is much smaller than $\sigma_B$; $Bias_{BA}$ is an average measurement deviation between the first and second body composition data, and an initial default value for the average measurement deviation is zero; if $\sigma_A$ is zero and $\sigma_B$ is not zero,

$$\widehat{x_B} = x_A = x_B - (x_B - x_A)$$

which is approximate to

$$\widehat{x_B} = x_A = x_B - Bias_{BA}$$

.

5. The data integration system (10) of the body composition analyzer as claimed in claim 4, wherein the data integration unit (45) includes a data determining logic (453) requesting measurement time points of the plural first and second body composition data and classifying the first and second body composition data in pairs and obtaining plural data according the sequence of the measurement time points; when the plural data are zero, and there is no the average measurement deviation, the average measurement deviation is zero; when the plural data are one, and there is no the average measurement deviation, the average measurement deviation is a difference be-

**EP 4 220 665 A1**

tween the first and second body composition data; when the plural data are smaller than or equal to three, the average measurement deviation is based on those with a relatively large amount of usage data; when the plural data are larger than three, the average measurement deviation is obtained by using a statistical method.

6. The data integration system (10) of the body composition analyzer as claimed in claim 5, wherein the data determining logic (453) has a predetermined interval capable of being set for requesting the measurement time points of the first and second body composition data within the predetermined range.

7. The data integration system (10) of the body composition analyzer as claimed in claim 4, wherein the computing unit (43) has a trendline calculational logic (431).

FIG. 1

431

trendline calculational logic

43

computing unit

41

40

47

database

computing device

display unit

45

data integration unit

451

analysis data

453

data determining logic

FIG. 2

FIG. 3

FIG. 4

Europäisches
Patentamt

European
Patent Office

Office européen
des brevets

## EUROPEAN SEARCH REPORT

Application Number

EP 23 15 1862

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/393158 A1 (KODAMA MIYUKI [JP] ET AL) 23 December 2021 (2021-12-23) * The whole document, in particular: Paragraphs: [0007], [0008], [0042] – [0059]. Figures: 1, 2, 5A and 5B. * ----- | 1-7 | INV. G16H50/30 G16H40/63 G01G1/00 A61B5/00 A61B5/0537 ADD. G01G19/50 |

TECHNICAL FIELDS SEARCHED (IPC)

G16H
G01G
A61B

The present search report has been drawn up for all claims

| Place of search | Date of completion of the search | Examiner |
|---|---|---|
| Munich | 28 April 2023 | Zacharias, Malte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 1862

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2023

| Patent document cited in search report | | Publication date | Patent family member(s) | | Publication date |
|---|---|---|---|---|---|
| US 2021393158 | A1 | 23-12-2021 | CN | 113556973 A | 26-10-2021 |
| | | | EP | 3936042 A1 | 12-01-2022 |
| | | | JP | 7214207 B2 | 30-01-2023 |
| | | | JP | 2020141863 A | 10-09-2020 |
| | | | US | 2021393158 A1 | 23-12-2021 |
| | | | WO | 2020179807 A1 | 10-09-2020 |

EPO FORM P0459

For more details about this annex : see Official Journal of the European Patent Office, No. 12/82